# EUROPÄISCHE PATENTSCHRIFT

(11) **EP 2 387 954 B1**
(45) Veröffentlichungstag und Bekanntmachung des Hinweises auf die Patenterteilung: **09.08.2017**
(21) Anmeldenummer: 11003949.2
(22) Anmeldetag: 13.05.2011
(51) Int. Cl.: A61B 17/04

(54) **Endoskopische Nähmaschine**
Endoscopic sewing machine
Machine à coudre endoscopique

(30) Priorität: 18.05.2010 DE 102010021273
(43) Veröffentlichungstag der Anmeldung: 23.11.2011
(73) Patentinhaber: Karl Storz GmbH & Co. KG, 78532 Tuttlingen (DE)
(72) Erfinder: Frings, Hermann-Josef, 52072 Aachen (DE); Krings, Manfred, 52080 Aachen (DE); Moik, Gunter, 52066 Aachen (DE)

(56) Entgegenhaltungen:
- WO-A1-01/01868
- DE-A1- 10 116 171
- DE-B3-102004 056 204
- FR-A- 920 113
- FR-A1- 2 924 917
- US-A- 4 440 171
- US-B1- 6 786 913

## Beschreibung

Die Erfindung betrifft eine endoskopische Nähmaschine mit einer Einrichtung zur Bevorratung und Führung des Fadens.

Die DE 101 16 171 A1 offenbart eine endoskopische Nähmaschine mit einem Gehäuse, das im Wesentlichen von einem zur Aufnahme von Antrieben für die Stichbildwerkzeuge dienenden Gehäuseoberteil und einem daran anschließenden Gehäuseschaft gebildet ist, der die Mittel zur Übertragung der von den Antrieben erzeugten Bewegungen auf die Stichbildewerkzeuge aufnimmt.

Diese umfassen zumindest eine von einer Nadelstange aufgenommene fadenführende Nadel und einen mit dieser zur Bildung von Stichen zusammenwirkenden Greifer, der von einer Greiferstange aufgenommen ist.

Die Frage der Bevorratung und Zuführung des Fadens von einem Fadenvorrat zum Öhr der Nadel ist in der DE 101 16 171 A1 ebenso wenig angesprochen, wie die Frage der Einstellung der zur Bildung der einzelnen Stiche erforderlichen Größe der Fadenspannung. Dies ist insoweit auch verständlich, als durch die Integration dieser Einrichtungen in eine endoskopische Nähmaschine diese wesentlich komplizierter und damit auch wesentlich störanfälliger würden. Gerade dies soll aber im Hinblick darauf, dass endoskopische Nähmaschinen überwiegend innerhalb des menschlichen oder tierischen Körpers eingesetzt werden und deshalb äußerst zuverlässig sein sollen, vermieden werden.

Bei der endoskopischen Nähmaschine nach der DE 101 16 171 A1, die eine sogenannte "einfädige Überwendlichnaht" bildet, wird zur Stichbildung ein Greifer verwendet, der nach dem Erfassen der durch die Nadel an der Unterseite des Nähgutes gebildeten Fadenschlinge entlang einer mehrdimensionalen Bewegungsbahn von der unterhalb des Nähgutes gelegenen, die Fadenschlinge erfassenden Position in eine oberhalb des Nähgutes gelegene Position bewegbar ist, in der das von der auf die Nähgutoberseite geführten Fadenschlinge gebildete Fadendreieck die Projektion der Nadelbahn umschließt.

Durch den häufigen Wechsel der Bewegungsrichtung des Fadens vom Verlassen des Fadenvorrates bis zum Einstich der Nadel in das auf der Oberseite des Nähgutes vom Greifer gebildete Fadendreieck durchläuft der Faden eine Vielzahl von bis zu 180° betragenden Umlenkstellen, was zu einer erheblichen Vervielfachung der im Faden wirksamen Zugkraft führt. Hierdurch werden sowohl die Nadelstange, als insbesondere auch die den Greifer aufnehmende Greiferstange einer radialen Belastung ausgesetzt, die ein Vielfaches der für den Sticheinzug notwendigen Fadenrückhaltekraft beträgt. Diese Belastungen insbesondere der Greiferstange, als aber auch der Nadelstange führen daher sehr schnell zu Verformungen der Nadel- und insbesondere der Greiferstange, die sehr häufig die Ursache für die Entstehung von Fehlstichen sind.

Der Erfindung liegt daher die Aufgabe zu Grunde, sowohl die auf die Greiferstange, als auch die auf die Nadelstange einwirkenden Fadenkräfte zu minimieren und hierdurch die Sicherheit der Stichbildung zu erhöhen.

Hierzu geht die Erfindung von der Erkenntnis aus, dass sich die im Bereich der Fadenspanneinrichtung erforderliche Fadenrückhaltekraft im Wesentlichen aus zwei Komponenten zusammensetzt, nämlich dem Betrag der nähtechnisch erforderlichen eigentlichen Fadeneinzugskraft und den zur Überwindung der Fadenreibung von der Stichbildestelle zur Fadenspanneinrichtung zu überwindenden Reibungskräften.

Der Erfindung liegt daher die Aufgabe zu Grunde, den Verlauf des Fadens von der Stichbildestelle zur Fadenspanneinrichtung so zu gestalten, dass die Fadenreibungskräfte weitestgehend minimiert werden.

Diese Aufgabe wird erfindungsgemäß dadurch gelöst, dass die Nadelstange zur Aufnahme und Führung des Fadens als Hohlkörper ausgebildet ist, und die Fadenspanneinrichtung in Bezug auf die Nadelstange derart angeordnet ist, dass der Faden zwischen der Fadenspanneinrichtung und seinem Eintritt in die Nadelstange im Wesentlichen geradlinig verläuft.

Durch die Maßnahme, die Nadelstange als den Faden aufnehmenden Hohlkörper auszubilden, und die Fadenspanneinrichtung in Bezug auf die Nadelstange derart anzuordnen, dass der Faden zwischen der Fadenspanneinrichtung und seinem Eintritt in die Nadelstange im Wesentlichen geradlinig verläuft, wird erreicht, dass die von der Fadenspanneinrichtung aufzubringende Fadenrückhaltekraft nur geringfügig größer dimensioniert werden muss, als die zur Gewährleistung des Sticheinzuges erforderliche Sticheinzugskraft. Da die erfindungsgemäße Ausbildung der Nadelstange als Hohlkörper die Möglichkeit bietet, den Faden wahlweise seitlich oder stirnseitig aus der Nadelstange heraus zu führen, entfallen für den Faden zwischen dem Eintritt in die Nadelstange bis zu dessen Austritt aus der Nadelstange sämtliche seither notwendigen Umlenkungen, sodass die noch wirksam werdenden Reibungskräfte auf ein äußerst geringes Minimum zurück geführt werden können.

Durch die FR 2924 917 A1 ist für das Befestigen einer als Prothese dienenden Gewebestruktur auf einem Körperteil eine Vorrichtung bekannt, bei der als Verbindungsmittel zu einer Schleife vorgeformte Formgedächtnis-Metallfaden verwendet werden. Die Vorrichtung zum Einbringen Metallfaden-Schleifen weist im Wesentlichen ein Gehäuse mit einem Griff und einer hohlen Stange auf. Mit der Stange ist ein drehbarer Zylinder verbunden, der mehrere achsparallele Durchgangsbohrungen enthält. Den Durchgangsbohrungen ist ein durch einen Schalthebel betätigbares Ausstoßmittel zugeordnet, das von einem nadelförmigen Stift gebildet ist. Dem Zylinder ist ein Fortschaltmechanismus zugeordnet, der bei jedem Betätigen des Schalthebels den Zylinder um ein bestimmtes Winkelmaß weiterdreht.

In die einzelnen Durchgangsbohrungen wird vor Gebrauch der Vorrichtung je ein aus Formgedächtnis-Material bestehender Fadenabschnitt eingeführt, wobei er eine an die Form der Durchgangsbohrung angepasste lineare Gestalt annimmt. Durch Betätigen des Schalthebels wird mittels des nadelförmigen Stiftes ein Fadenabschnitt aus dem Zylinder ausgestoßen und dabei in die Prothese sowie das darunter liegende Körperteil eingeschoben. Nachdem der Fadenabschnitt die Durchgangsbohrung verlassen hat, kann er sich mit der jetzt wirksam werdenden Rückstellkraft der Formgedächtnisfunktion in seine ursprüngliche Schleifenform zurückverformen, wodurch er die Prothese mit dem Körperteil verbindet.

Da diese Vorrichtung weder eine hin- und herbewegbare Nadelstange mit einer fadenführenden Nadel, noch einen mit dieser zur Bildung von Überwendlichstichen zusammenwirkenden Greifer aufweist, sondern bei ihr der Metallfaden allein aufgrund der ausreichend großen Eigensteifigkeit in die Prothese und das Körperteil eingestochen werden kann und das Verformen des Metallfadens zu einer Schleife nur durch die Rückstellkraft des Formgedächtnis-Materials erfolgt, ist die bekannte Vorrichtung gegenüber Nähmaschinen gattungsfremd und kann daher keinen Beitrag zur Lösung der erfindungsgemäßen Aufgabe geleistet haben.

Durch die US 6 786 913 B1 ist eine chirurgische Schleifenbildevorrichtung bekannt, mit der ein für chirurgische Nähte verwendbarer Draht ohne Mithilfe einer Nadel durch zwei miteinander zu verbindende Gewebeabschnitte hindurchgestoßen, danach eine Schleife gebildet und diese durch Verdrillen gegen Aufziehen gesichert wird. Da auch diese Vorrichtung weder eine hin- und herbewegbare Nadelstange mit einer fadenführenden Nadel, noch einen mit dieser zur Bildung von Überwendlichstichen zusammenwirkenden Greifer aufweist, ist auch sie gegenüber Nähmaschinen gattungsfremd und kann daher ebenfalls keinen Beitrag zur Lösung der erfindungsgemäßen Aufgabe geleistet haben.

*Durch die* US 4 440 171 A *sind ferner ein Chirurgischer Nähapparat und eine Methode zur Halterung eines Schiffchens bekannt. Der Nähapparat weist eine Nadelstange auf, die an ihrem Ende an einem gehäusefest angeordneten Nadelstangenhalter befestigt ist. Am anderen Ende der Nadelstange ist ein Fadenführungsstück befestigt, das eine quer zur Längsachse der Nadelstange gerichtete Bogennadel trägt. An der konvexen Seite der Bogennadel ist eine in einem Öhr mündende offene Rille für die Aufnahme des Nadelfadens ausgebildet. Der Nadelfadenvorrat ist in einem flachen spulenförmigen Fadenspeicher aufgenommen, von wo aus er entgegen der Richtung der Bogennadel zu einer Fadenspanneinrichtung verläuft. Von dort aus wird der Nadelfaden um 180° umgelenkt und verläuft sodann durch eine Fadenführungsnut im Wesentlichen parallel zur Längsachse der Nadelstange an deren Außenseite entlang zum Fadenführungsstück und von dort um 90° abgewinkelt durch die offene Rille der Bogennadel hindurch zum Nadelöhr. Der Bogennadel ist ein Schiffchen zugeordnet, das in einem Schiffchenhalter frei beweglich ist. Der Schiffchenhalter ist an einer in axialer Richtung verschiebbar gelagerten unverdrehbaren Stange befestigt. Die Stange ist mit einem Handgriff versehen, mit dessen Hilfe sie und damit das Schiffchen auf die Bogennadel zu- und von ihr wegbewegt werden kann.*

*Bei dem bekannten Nähapparat durchläuft der Nadelfaden auf seinem Weg vom Fadenspeicher zur Bogennadel also zwei markante Umlenkstellen, welche erhöhte Fadenreibungskräfte hervorrufen. Da ferner die Nadelstange fest mit dem Apparategehäuse verbunden ist und darüber hinaus auch aus massivem Stangenmaterial besteht, kann daher der bekannte Nähapparat keinen Beitrag zur Lösung der erfindungsgemäßen Aufgabe geleistet haben.*

Bei der erfindungsgemäßen endoskopischen Nähmaschine kann eine besonders einfache und eine glatte Führungsfläche aufweisende Führung für den Faden innerhalb der Nadelstange durch Verwendung eines innerhalb der hohlen Nadelstange angeordneten Führungselementes erreicht werden, das vorzugsweise aus einem Kunststoff besteht.

Alternativ zu dem stirnseitigen Austritt des Fadens aus der Nadelstange kann ein seitlicher Austritt dadurch erreicht werden, wenn die Nadelstange im Bereich des Befestigungsmittels für die Nadel eine nach außen und vorzugsweise schräg nach abwärts gerichtete Austrittsöffnung für den Faden aufweist.

Sofern die zur Verwendung kommende Nadel eine mit einer Fadenrille und einem Flachkolben versehene Nadel ist, ist sowohl eine Führung als auch ein Schutz des Fadens gegen äußere Einflüsse erreichbar, wenn die Längsnut zusammen mit der Fadenrille der Nadel einen Fadenkanal bildet und der Faden an dessen offener Stirnseite aus dem Fadenkanal austritt.

In einer weiteren Ausgestaltung der Erfindung kann die Nadel als Kanüle ausgebildet sein, wobei es zur Erhöhung der Sicherheit des Erfassens der Fadenschleife durch die Greiferspitze vorteilhaft ist, wenn die Kanüle zur Reduzierung des seitlichen Abstandes zwischen dieser und der Bewegungsbahn der Greiferspitze im üblichen Bereich des Nadelöhrs eine seitliche Ausnehmung für die Greiferspitze aufweist.

Hierbei kann die Kanüle in diesem Bereich einen schrägen Anschliff aufweisen, wodurch eine zur Seite gerichtete ovale Austrittsöffnung für den Faden entsteht. Die Maßnahme, den Fadenvorrat innerhalb einer Aufnahmevorrichtung aufzunehmen und diese beispielsweise mittels einer Schraubverbindung mit der Nadelstange lösbar zu verbinden, führt einerseits zu einem relativ kurzen und umlenkungsfreien Fadenweg und bietet andererseits die Möglichkeit, zunächst die Nadelstange und die leere Aufnahmevorrichtung gemeinsam zu sterilisieren und diese nach Befüllung und Einfädeln der Nadel als einsatzfähige Baugruppe zu lagern und diese im Bedarfsfall als Baugruppe in die Maschine einzusetzen.

Dabei ist es zum Einsatz von vorgefüllten Fadenspulen vorteilhaft, die Aufnahmevorrichtung für den Fadenvorrat von einem zweiteiligen Gehäuse zu bilden und dessen Basisteil mit einer zylinderförmigen Aufnahme, sowie mit einem Zentrierzapfen für die Fadenspule zu versehen.

Einem Schwergang der Fadenspule beim Abziehen des Fadens kann dadurch vorgebeugt werden, wenn an dem Zentrierzapfen eine Auflagefläche für die Fadenspule ausgebildet ist, die einen axialen Abstand zur Bodenfläche der Aufnahme aufweist.

Um beim Wechsel der Nadelstange deren Antriebsverbindung zum diese bewegenden Pleuel nicht stets im Rahmen einer Art Demontage der Maschine lösen zu müssen, ist die Nadelstange innerhalb einer von dem Pleuel angetriebenen Hülse angeordnet und ist mit dieser lösbar verbunden. Die Bewegung des Pleuels wird hierbei von der Hauptwelle der Maschine abgeleitet. Hierzu ist die Hülse über ein übliches Drehgelenk mit dem Pleuel verbunden, während die Verbindung zwischen der Nadelstange und der Hülse mittels einer lösbaren Schnellverbindung erfolgt. Diese kann in einfacher und sicherer Weise von einem Bajonettverschluss gebildet sein, dessen Festteil mit der Hülse, und dessen Losteil mit der Nadelstange verbunden ist. Damit kann die Nadelstange praktisch mit einem Griff der Maschine entnommen und ebenso leicht wieder in die Maschine eingesetzt werden.

Sowohl zum radialen als auch zum axialen justieren der Nadelstange 5 wird das Festteil 30 des Bajonettverschlusses relativ zum Gelenkpunkt zwischen dem Pleuel 27 und der Hülse 28 verstellt. Hierzu kann der Gelenkpunkt zwischen dem Pleuel und der Hülse als Klemmvorrichtung zur Aufnahme der Hülse ausgebildet sein.

Um sowohl den Fadenvorrat, als auch den von der Fadenspule zur Nadelstange führenden Bereich des Fadens gegen äußere Einflüsse zu schützen, ist zusätzlich zum Fadenvorrat auch die Fadenspanneinrichtung innerhalb der Aufnahmevorrichtung angeordnet.

Eine einfache Ausbildung für die Fadenspanneinrichtung wird dadurch erreicht, dass diese im Wesentlichen von zwei parallel zueinander und übereinander angeordneten Druckstücken gebildet ist, wobei das obere Druckstück kraftschlüssig auf dem unteren Druckstück aufliegt, und der den Kraftschluss bewirkenden Feder zur Veränderung der Druckkraft ein Stellmittel zugeordnet ist, dessen Handhabe aus der Aufnahmevorrichtung heraus ragt.

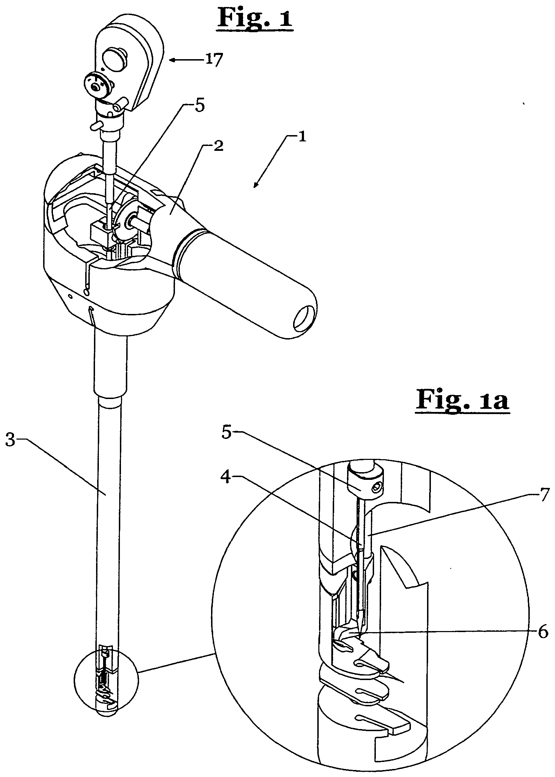

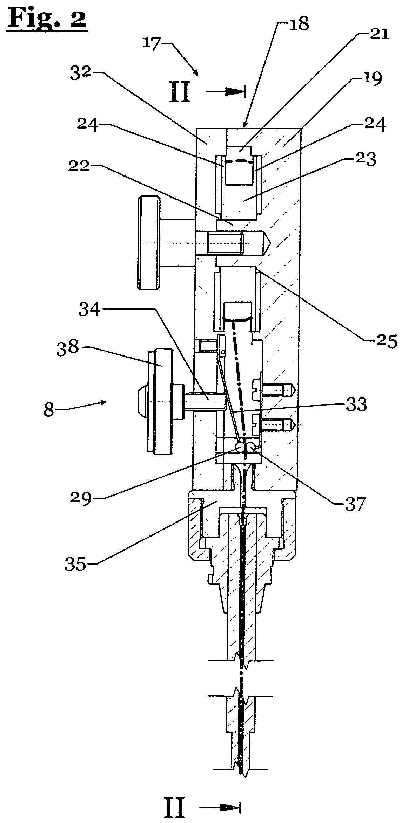

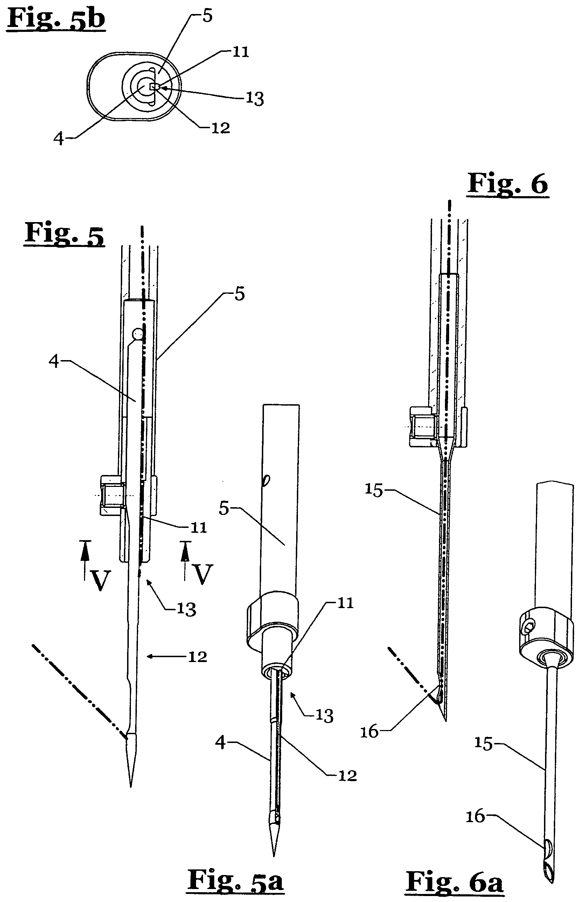

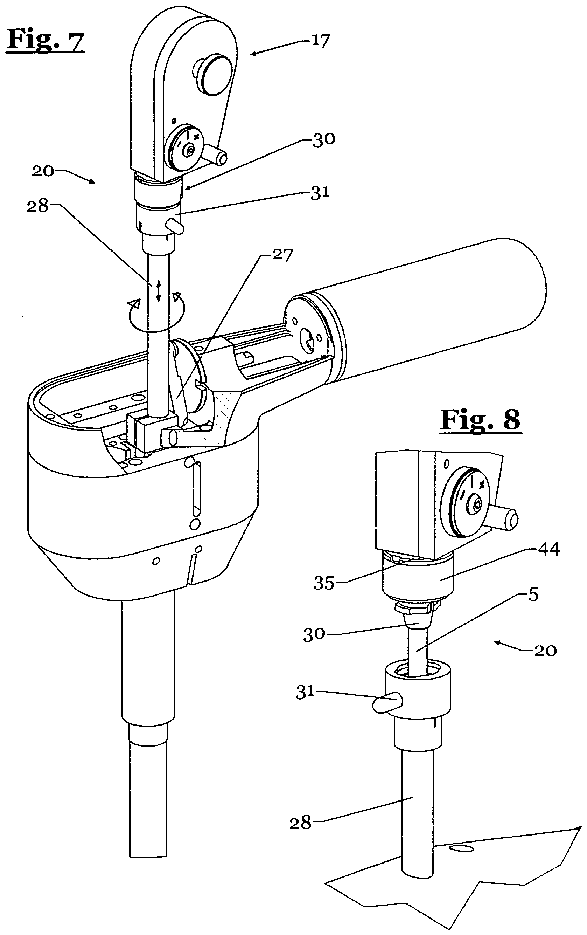

Weitere Einzelheiten und Vorteile der Erfindung ergeben sich anhand der nachfolgenden Beschreibung eines in der Zeichnung dargestellten Ausführungsbeispieles der Erfindung. Es zeigt:
Fig. 1:
   Eine schaubildliche Darstellung einer endoskopischen Nähmaschine;
Fig. 1 a:
   Eine vergrößerte Darstellung des Stichbildebereiches der Fig.1;
Fig. 2:
   Eine Schnittdarstellung der Aufnahmevorrichtung für den Fadenvorrat und die Fadenspanneinrichtung,
Fig. 3:
   Einen Schnitt nach Linie II-II der Fig. 2;
Fig. 3 a:
   Eine vergrößerte Darstellung des Bereiches der Austrittsöffnung der Nadelstange;
Fig. 4:
   Eine Schnittdarstellung des Verbindungsbereiches der Nadelstange mit der Aufnahmevorrichtung für den Fadenvorrat und der Fadenspanneinrichtung;
Fig. 5:
   Eine Schnittdarstellung einer ersten Ausführung der Nadelstange und der Nadel;
Fig. 5 a:
   Eine Frontansicht der ersten Ausführung der Nadelstange und der Nadel;
Fig. 5 b:
   Einen Schnitt nach Linie 5-5 der Fig. 5;
Fig. 6:
   Eine Schnittdarstellung einer zweiten Ausführung der Nadelstange und der Kanüle;
Fig. 6 a:
   Eine Frontansicht der zweiten Ausführung der Nadelstange und der Kanüle;
Fig. 7:
   Eine der Fig. 1 entsprechende Darstellung mit der Aufnahmevorrichtung und dem Bajonettverschluss;
Fig. 8:
   Eine vergrößerte Darstellung einer Einzelheit des Bajonettverschlusses;

In Fig. 1 ist eine schematische Darstellung eines Gehäuses 1 einer endoskopischen Nähmaschine gezeigt, das ein Gehäuseoberteil 2 zur Aufnahme von Antrieben für die Stichbildewerkzeuge, sowie einen sich an das Gehäuseoberteil anschließenden Gehäuseschaft 3 zur Aufnahme von Mitteln zur Übertragung der von den Antrieben erzeugten Bewegungen auf die Stichbildewerkzeuge aufweist. Diese bestehen im Wesentlichen aus einer einen Faden führenden Nadel 4, einer diese aufnehmenden Nadelstange 5, sowie einem mit der Nadel 4 zur Bildung von Stichen zusammenwirkenden Greifer 6, der von einer Greiferstange 7 aufgenommen ist. Die Greiferstange 7 ist im Gehäuseschaft 3 verschieb- und drehbar gelagert, sodass der Greifer 6 nach dem Erfassen der durch die Nadel 4 an der Nähgutunterseite gebildeten Fadenschlinge entlang einer mehrdimensionalen Bewegungsbahn von der unterhalb des Nähgutes gelegenen, die Fadenschlinge erfassenden Position in eine oberhalb des Nähgutes gelegene Position bewegbar ist, in der das von der auf die Nähgutoberseite geführten Fadenschlinge gebildete Fadendreieck die Projektion der Nadelbahn umschließt.

Die prinzipielle Ausbildung der Stichbildewerkzeuge und deren Wirkungsweise kann dabei der Wirkungsweise der endoskopischen Nähmaschine gemäß DE 101 16 171 A1 entsprechen, die dazu dient, eine Überwendlichnaht unter Verwendung einer dem Stichtyp 501 entsprechenden Stichart zu bilden.

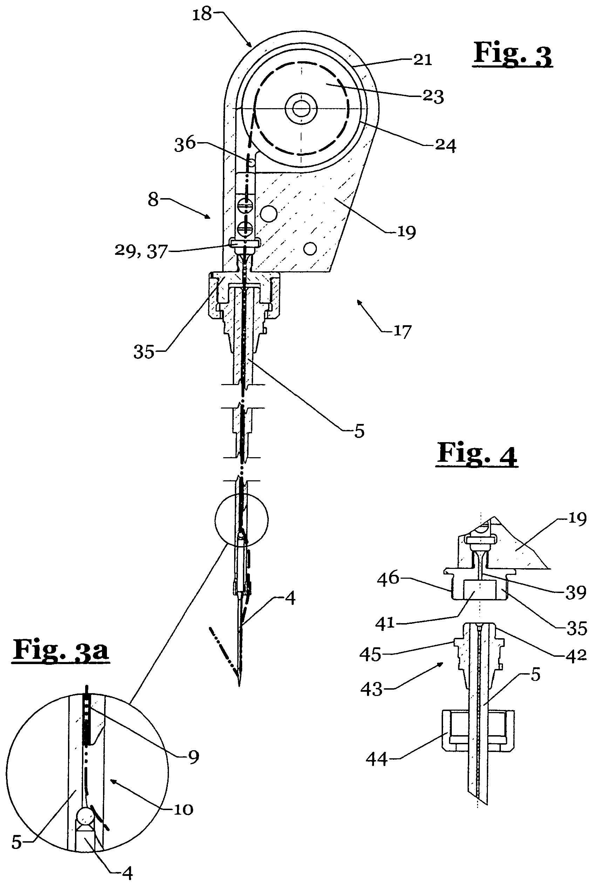

Um beim Sticheinzug ein Nachziehen von Faden vom Fadenvorrat zu vermeiden, ist eine Fadenspanneinrichtung 8 (Fig. 2, 3) vorgesehen, deren Rückhaltekraft für den Faden größer als die Fadeneinzugskraft ist.
Die Nadelstange 5 ist zur Aufnahme und Führung des Fadens als Hohlkörper ausgebildet, wobei die Fadenspanneinrichtung 8 in Bezug auf die Nadelstange 5 derart angeordnet ist, dass der Faden zwischen dem Austritt aus der Fadenspanneinrichtung 8 und dem Eintritt in die Nadelstange 5 im Wesentlichen geradlinig verläuft.

Da durch diese Relativlage der Fadenspanneinrichtung 8 zur als Hohlkörper ausgebildeten Nadelstange 5 alle seither auf dem Weg des Fadens vom Fadenvorrat zur Nadel 4 vorhandenen Fadenumlenkungen entfallen, reduziert sich einerseits der Reibungswiderstand für den Faden und andererseits übernimmt die Nadelstange 5 zusammen mit der Nadel 4 die Funktion des seither erforderlichen Fadenhebels.

Innerhalb der Nadelstange 5 ist ein diese durchsetzendes Führungselement 9 (Fig. 3a) für den Faden angeordnet, das vorzugsweise schlauchförmig ausgebildet ist und aus Kunststoff gefertigt sein kann. An der Nadelstange 5 ist ferner im Bereich des üblichen Befestigungsmittels für die Nadel 4 eine nach außen und vorzugsweise schräg nach abwärts gerichtete Querbohrung vorgesehen, die als Austrittsöffnung 10 für den Faden aus der Nadelstange 5 dient.

Alternativ hierzu kann die Nadelstange 5 im Bereich der Befestigungsmittel für die Nadel 4 eine zur Aufnahme des Fadens dienende Längsnut 11 (Fig. 5, 5a, 5b) aufweisen.

Sofern eine mit einem Flachkolben und mit einer Fadenrille 12 versehene Nadel 4 verwendet wird, kann die Längsnut 11 zusammen mit der Fadenrille 12 der Nadel 4 einen Fadenkanal 13 zur Aufnahme des Fadens bilden, und dieser an der offenen Stirnseite des Fadenkanals 13 aus diesem herausgeführt werden.

In einer weiteren Gestaltung der Nadel 4 (Fig. 6, 6a) ist diese als Kanüle 15 ausgebildet und im Bereich des Fadenaustrittes mit einer seitlichen Ausnehmung 16 für die Spitze des Greifers 6 versehen, wodurch die Sicherheit der Erfassung der Nadelfadenschlinge durch den Greifer 6 erhöht wird.

Zur Aufnahme des Fadenvorrates ist eine Aufnahmevorrichtung 17 (Fig. 2, 3) vorgesehen, die von einem zweiteiligen Gehäuse 18 gebildet ist und ein Basisteil 19 aufweist. Am Basisteil 19 ist eine zylinderförmige Aufnahme 21 für eine den Fadenvorrat aufnehmende Fadenspule 23 ausgebildet. Dabei umschließt die Innenwandung der Aufnahme 21 die in diese eingelegte Fadenspule 23, wobei der Durchmesser der Flansche 24 der Fadenspule 23 geringfügig -vorzugsweise nur einige zehntel Millimeter- kleiner als der Innendurchmesser der Aufnahme 21 ist. Damit ist sichergestellt, dass der Faden selbst dann, wenn er sich von der Fadenspule 23 lösen sollte, sich nicht zwischen dieser und der Innenwandung der Aufnahme 21 einklemmt. Zur Führung der Fadenspule 23 innerhalb der Aufnahme 21 ist in dieser ein Zentrierzapfen 22 vorgesehen, an dem auch eine ringförmige Auflagefläche 25 für die Fadenspule 23 ausgebildet ist. Zur Minimierung des Reibmomentes der Fadenspule 23 beim Abzug des Fadens ist der Außendurchmesser der Auflagefläche 25 nur unwesentlich größer als der Durchmesser der Bohrung in der Fadenspule 23. Zum Verschließen der Aufnahmevorrichtung 17 und damit des zweiteiligen Gehäuses 18 ist ein Deckel 32 vorgesehen, dessen Form der Form des Basisteiles 19 entspricht. Der Deckel 32 ist in an sich bekannter Weise mittels einer Schraubverbindung mit dem Basisteil 19 lösbar verbunden und wird zum Einlegen der befüllten Fadenspule 23 in die Aufnahme 21 abgenommen.

Zur Erzielung und Einstellung der Fadenspannung ist innerhalb des Gehäuses 18 auch die Fadenspanneinrichtung 8 angeordnet, die im Wesentlichen von zwei parallel zueinander und übereinander angeordneten Druckstücken 29, 37 gebildet ist, wobei der Faden zwischen diesen hindurch bewegt wird. Die Druckstücke 29, 37 können die Form von kleinen zylindrischen Walzen aufweisen, oder können von Zylinderstiften gebildet sein. Gegebenenfalls können die Zylinderstifte zur Reduzierung des Reibungskoeffizienten mit einem Kunststoff ummantelt sein, oder aus Kunststoff bestehen. Die Druckstücke 29, 37 liegen kraftschlüssig aufeinander auf, wobei das obere Druckstück 29 kraftschlüssig auf dem unteren Druckstück 37 aufliegt. Hierbei wird der Kraftschluss durch eine auf dem oberen Druckstück 29 aufliegende Biegefeder 33 erzeugt. Bei entsprechender Anordnung kann anstelle der Biegefeder 33 auch eine Druck- oder Zugfeder den Kraftschluss erzeugen.

Zur Veränderung der auf das obere Druckstück 29 einwirkenden Kraft und somit zur Veränderung der Rückhaltekraft der Fadenspanneinrichtung 8 weist diese ein Stellmittel 34 auf, das zwischen den Auflagepunkten der Biegefeder 33 auf diese einwirkt. Das Stellmittel 34 ist mit einer aus dem Gehäuse herausragenden Handhabe 38 versehen, sodass bei deren Betätigung die Biegefeder 33 verformt und damit die Auflagekraft des oberen Druckstückes 29 auf den sich zwischen den Druckstücken 29, 37 befindlichen Faden entsprechend verändert wird.

Zum Austritt des Fadens aus der geschlossenen Aufnahmevorrichtung 17 ist am Basisteil 19 des Gehäuses 18 eine Fadenführung 36 vorgesehen, die in einfacher Weise von einem quer zur Abzugsrichtung des Fadens gerichteten Stift gebildet ist. Ferner sind die Anordnung der Aufnahme 21, des in dieser angeordneten Zentrierzapfens 22, sowie der Fadenführung 36 so getroffen, dass der Abzug des Fadens in der Gebrauchslage der Maschine im Wesentlichen geradlinig nach abwärts erfolgt.

Der Faden verläuft daher beim Fadenabzug zunächst innerhalb des Gehäuses 18 von der Fadenspule 23 über die Fadenführung 36 zwischen den Druckstücken 29, 37 hindurch zu einem an der unteren Stirnseite des Gehäuses 18 angeordneten und an dessen Basisteil19 befestigten Verbindungsstück 35. Das Verbindungsstück 35 ist an seinem freien Ende mit einem Außengewinde 46 versehen und weist eine axial gerichtete Bohrung 39 auf, die sich zum Durchtritt des Fadens einerends bis ins Innere des Basisteiles 19 erstreckt und anderenendes in eine Stufenbohrung 41 mündet. Die Stufenbohrung 41 dient zur Aufnahme eines Kopfes 42 eines Zwischenstückes 43, das am oberen Ende der Nadelstange 5 angeordnet und an dieser befestigt ist. Auf der Nadelstange 5 ist eine mit dem Außengewinde 46 des Verbindungsstückes 35 zusammenwirkende Überwurfmutter 44 verschieblich angeordnet, die zum Verbinden der Nadelstange 5 mit dem Verbindungsstück 35 einen am Zwischenstück 43 ausgebildeten Bund 45 hintergreift. Im gefügten Zustand von Nadelstange 5 und Verbindungsstück 35 ragt der mit der hohlen Nadelstange 5 verbundene Kopf 42 in die Stufenbohrung 41 des Verbindungsstückes 35, wobei dessen Bohrung 39 mit dem innerhalb der hohlen Nadelstange 5 angeordneten Führungselement 9 für den Faden fluchtet.

Hierdurch verläuft der Faden von der Fadenspule 23 über die Fadenführung 36 und die Fadenspanneinrichtung 8 sowie durch das Verbindungsstück 35 hindurch bis zum Eintritt in die Nadelstange 5 und von da bis zum Austritt aus der Nadelstange 5 nahezu umlenkungsfrei und damit nahezu geradlinig.

Die Nadelstange 5 ist innerhalb einer von einem Pleuel 27 angetriebenen Hülse 28 angeordnet und mit dieser lösbar verbunden. Die Bewegung des Pleuels 27 wird von der Hauptwelle der Maschine abgeleitet. Hierzu ist die Hülse 28 über ein übliches Drehgelenk mit dem Pleuel 27 verbunden, während die Verbindung zwischen der Hülse 28 und der Nadelstange 5 von einem Bajonettverschluss 20 gebildet ist, dessen Festteil 30 mit der Hülse 28, und dessen Losteil 31 mit der Nadelstange 5 verbunden ist.

Sowohl zum radialen als auch zum axialen justieren der Nadelstange 5 wird das Festteil 30 des Bajonettverschlusses relativ zum Gelenkpunkt zwischen dem Pleuel 27 und der Hülse 28 verstellt. Hierzu kann der Gelenkpunkt zwischen dem Pleuel 27 und der Hülse 28 als Klemmvorrichtung zur Aufnahme der Hülse 28 ausgebildet sein. Innerhalb dieser Klemmvorrichtung wird die Hülse 28 bei der Montage einmalig so positioniert, dass die Nadel des Nadelstangen-Systems hinsichtlich ihrer axialen und radialen Lage richtig steht. Sofern diese Positionierung der Nadel bei allen Nadelstangen richtig vorgenommen ist, kann die Nadelstange 5 durch Lösen des Bajonettverschlusses 20 zusammen mit der von ihr aufgenommenen Nadel 4 mit einem Griff von der Maschine gelöst und dieser entnommen werden. Wird hierbei die Überwurfmutter 44 nicht gelöst, können die Nadelstange 5 und die Aufnahmevorrichtung 17 mit der Fadenspule 23 und der Fadenspanneinrichtung 8 mit nur einem einzigen Handgriff von der Maschine gelöst und dieser entnommen werden.

Die Aufnahmevorrichtung 17 und die Nadelstange 5 bilden damit ein sowohl die Nadel 4 und den Fadenvorrat, als auch die Fadenspanneinrichtung aufnehmendes "Nadelstangen-System" das bei seiner Herstellung mittels einer Einstelllehre justiert wird und als unmittelbar einsatzfähiges Aggregat vorrätig gelagert werden kann.

Umgekehrt können die Aufnahmevorrichtung 17 für die Fadenspule 23 und die Fadenspanneinrichtung 8 außerhalb der Maschine mit einer neuen Fadenspule 23 befüllt, der Anfangsfaden durch die Fadenspanneinrichtung 8 und das Verbindungsstück 35 hindurch unmittelbar bis zum Kopf 42 der Nadelstange 5 geführt und von da innerhalb der Nadelstange 5 bzw. innerhalb des Führungselementes 9 bis zum Öhr der Nadel geführt und auch eingefädelt werden.

Da die im Rahmen der Fertigung der Nadelstangen-Systeme vorgenommene Justierung sowohl der axialen als auch der radialen Lage der Nadelstange bei allen Nadelstangen-Systemen gleich ist und sich diese beim Wechsel der Nadelstange nicht verändert, können die so vormontierten Nadelstangensysteme als "nähbereite Einheit" gelagert und ohne jegliches weiteres Justieren in jede Maschine eingesetzt und mit einem Handgriff in dieser fixiert werden.

Dies bietet die Möglichkeit, mit unterschiedlichen Nadeln und / oder unterschiedlichen Fäden bestückte "nähbereite Nadelstangensysteme" vorrätig zu halten, um sie im Bedarfsfall schnell in die Maschine einsetzen und mit dem Nähen beginnen zu können.

## Patentansprüche

1. Endoskopische Nähmaschine, aufweisend ein Gehäuse (1) mit einem Gehäuse-oberteil (3) zur Aufnahme von Antrieben für die Stichbildewerkzeuge, einer einen Fadenvorrat aufnehmenden Fadenspule, einen sich an das Gehäuseoberteil anschließenden Gehäuseschaft zur Aufnahme von Mitteln zur Übertragung der von den Antrieben erzeugten Bewegungen auf die Stichbildewerkzeuge, die mindestens eine im Gehäuseschaft geführte Nadelstange (5) mit einer fadenführenden Nadel (4) und einen mit dieser zur Bildung von Stichen zusammen wirkenden Greifer (6) aufweisen, der von einer Greiferstange (7) aufgenommen ist, **dadurch gekennzeichnet, dass**, die Nadelstange (5) zur Aufnahme und Führung des Fadens als Hohlkörper ausgebildet ist, und die zwischen der den Fadenvorrat aufnehmenden Fadenspule (23) und der Nadelstange (5) angeordneten Fadenleitelemente (36,8,35)(36)(8)(35)) auf die Nadelstange (5) derart angeordnet sind, dass der Faden zwischen dem Austritt aus einer Aufnahmevorrichtung (17) für die Fadenspule (23) und dem Eintritt in die Nadelstange (5) geradlinig verläuft.

2. Endoskopische Nähmaschine nach Anspruch 1, **dadurch gekennzeichnet, dass** innerhalb der Nadelstange (5) ein diese durchsetzendes Führungselement (9) für den Faden angeordnet ist.

3. Endoskopische Nähmaschine nach Anspruch 1, **dadurch gekennzeichnet, dass** die Nadelstange (5) im Bereich der Befestigungsmittel für die Nadel (4) eine nach außen und vorzugsweise schräg nach abwärts gerichtete Austrittsöffnung (10) für den Faden aufweist,

4. Endoskopische Nähmaschine nach Anspruch 1, **dadurch gekennzeichnet, dass** die Nadelstange (5) im Bereich der Befestigungsmittel für die Nadel (4) eine zur Aufnahme des Fadens dienende Längsnut (11) aufweist.

5. Endoskopische Nähmaschine nach Anspruch 4 mit einer einen Flachkolben mit einer Fadenrille (12) aufweisenden Nadel (4), **dadurch gekennzeichnet, dass** die Längsnut (11) zusammen mit der Fadenrille (12) der Nadel (4) einen Fadenkanal (13) bildet, und der Faden an dessen offener Stirnseite aus dem Fadenkanal (13) austritt.

6. Endoskopische Nähmaschine nach Anspruch 1, **dadurch gekennzeichnet, dass** die Nadel (4) als Kanüle (15) ausgebildet ist und im üblichen Bereich des Öhrs eine seitliche Ausnehmung (16) für die Spitze des Greifers (6) aufweist.

7. Endoskopische Nähmaschine nach Anspruch 1 **dadurch gekennzeichnet dass** der Fadenvorrat innerhalb einer Aufnahmevorrichtung (17) aufgenommen ist und diese lösbar mit der Nadelstange (5) verbindbar ist.

8. Endoskopische Nähmaschine nach Anspruch 7, **dadurch gekennzeichnet dass** die Aufnahmevorrichtung (17) für den Fadenvorrat von einem zweiteiligen Gehäuse (18) gebildet ist, dessen Basisteil (19) eine zylinderförmige Aufnahme (21) mit einem Zentrierzapfen (22) für die Fadenspule (23) aufweist.

9. Endoskopische Nähmaschine nach Anspruch 7, **dadurch gekennzeichnet, dass** der Innendurchmesser der Aufnahme (21) für die Fadenspule (23) nur einige Zehntel Millimeter größer als der Außendurchmesser der Flansche (24) der Fadenspule (23) ist.

10. Endoskopische Nähmaschine nach Anspruch 8, **dadurch gekennzeichnet, dass** an dem Zentrierzapfen (22) eine Auflagefläche (25) für die Fadenspule (23) ausgebildet ist, die einen axialen Abstand zur Bodenfläche (26) der Aufnahme (21) aufweist.

11. Endoskopische Nähmaschine nach Anspruch 1, **dadurch gekennzeichnet, dass** die Nadelstange (5) innerhalb einer von einem Pleuel (27) angetriebenen Hülse (28) angeordnet und lösbar mit der Hülse verbindbar ist.

12. Endoskopische Nähmaschine nach Anspruch 11 **dadurch gekennzeichnet dass** die Verbindung zwischen der Hülse (28) und der Nadelstange (5) von einem Bajonettverschluss (20) gebildet ist, dessen Festteil (30) mit der Hülse (28), und dessen Losteil (31) mit der Nadelstange (5) verbunden ist.

13. Endoskopische Nähmaschine nach Anspruch 7 **dadurch gekennzeichnet, dass** die Fadenspanneinrichtung (8) innerhalb der Aufnahmevorrichtung (17) für den Fadenvorrat angeordnet ist, und von zwei parallel zueinander und übereinander angeordneten Druckstücken (29, 37) gebildet ist, wobei das obere Druckstück (29) kraftschlüssig auf dem unteren Druckstück (37) aufliegt.

14. Endoskopische Nähmaschine nach Anspruch 13 **dadurch gekennzeichnet, dass** der Kraftschluss durch eine auf dem oberen Druckstück (29) aufliegende Blatt - oder Druckfeder (33) gebildet wird.

15. Endoskopische Nähmaschine nach Anspruch 14, **dadurch gekennzeichnet, dass** der Blatt- oder Druckfeder (33) zur Veränderung ihrer Druckkraft ein Stellmittel (34) zugeordnet ist, dessen Handhabe (35) außerhalb des Gehäuses (1) angeordnet ist.

16. Endoskopische Nähmaschine nach Anspruch 8, **dadurch gekennzeichnet, dass** die Aufnahmevorrichtung (17) mittels eines Verbindungsstückes (35) mit der Nadelstange (5) verbunden ist, und das Verbindungsstück (35) gleichzeitig zur Führung des Fadens dient.

17. Endoskopische Nähmaschine nach Anspruch 16, **dadurch gekennzeichnet, dass** das Verbindungsstück (35) in Bezug auf die Aufnahmevorrichtung (17) und die Nadelstange (5) derart angeordnet sind, dass der Faden umlenkungsfrei von der Aufnahmevorrichtung (17) in das Verbindungsstück (35) einführbar ist.

## Claims

1. Endoscopic suturing machine having a housing (1) with a housing upper part (3) for receiving drives for the stitch-forming tools, a thread spool for receiving a thread supply, a housing shaft adjoining the housing upper part and receiving means by which the movements generated by the drives are transmitted to the stitch-forming tools, the at least one needle bar (5) guided in the housing shaft with a thread-guiding needle (4), and a gripper (6) which interacts with this to form stitches and is received by a gripper bar (7), **characterized in that** the needle bar (5) for receiving and guiding the thread is designed as a hollow body, and the thread-carrying elements (36, 8, 35) arranged between the thread spool (23), receiving the thread supply, and the needle bar (5) are arranged on the needle bar (5) in such a way that the thread runs in a straight line between the exit from a receiving device (17) for the thread spool (23) and the entry into the needle bar (5).

2. Endoscopic suturing machine according to Claim 1, **characterized in that** a guide element (9) for the thread is arranged inside the needle bar (5) and extends through the latter.

3. Endoscopic suturing machine according to Claim 1, **characterized in that** the needle bar (5), in the area of the fastening means for the needle (4), has an outlet opening (10) directed outwards and preferably obliquely downwards for the thread.

4. Endoscopic suturing machine according to Claim 1, **characterized in that** the needle bar (5), in the area of the fastening means for the needle (4), has a longitudinal groove (11) serving to receive the thread.

5. Endoscopic suturing machine according to Claim 4 with a needle (4) having a flat shank with a thread slot (12), **characterized in that** the longitudinal groove (11), together with the thread slot (12) of the needle (4), forms a thread channel (13), and the thread exits from the thread channel (13) at the open front face thereof.

6. Endoscopic suturing machine according to Claim 1, **characterized in that** the needle (4) is designed as a cannula (15) and, in the usual area of the needle eye, has a lateral recess (16) for the tip of the gripper (6).

7. Endoscopic suturing machine according to Claim 1, **characterized in that** the thread supply is received inside a receiving device (17), and the latter is releasably connectable to the needle bar (5).

8. Endoscopic suturing machine according to Claim 7, **characterized in that** the receiving device (17) for the thread supply is formed by a two-part housing (18), of which a base part (19) has a cylindrical seat (21) with a centring pin (22) for the thread spool (23).

9. Endoscopic suturing machine according to Claim 7, **characterized in that** the internal diameter of the seat (21) for the thread spool (23) is only a few tenths of a millimetre larger than the external diameter of the flanges (24) of the thread spool (23).

10. Endoscopic suturing machine according to Claim 8, **characterized in that** a bearing surface (25) for the thread spool (23) is formed on the centring pin (22), which bearing surface (25) is at an axial distance from the bottom surface (26) of the seat (21).

11. Endoscopic suturing machine according to Claim 1, **characterized in that** the needle bar (5) is arranged inside a sleeve (28) driven by a connecting rod (27) and is releasably connectable to the sleeve.

12. Endoscopic suturing machine according to Claim 11, **characterized in that** the connection between the sleeve (28) and the needle bar (5) is formed by a bayonet catch (20), of which a fixed part (30) is connected to the sleeve (28) and of which a movable part (31) is connected to the needle bar (5).

13. Endoscopic suturing machine according to Claim 7, **characterized in that** the thread tensioning means (8) is arranged inside the receiving device (17) for the thread supply and is formed by two pressure pieces (29, 37) arranged parallel to each other and one on top of the other, wherein the upper pressure piece (29) lies on the lower pressure piece (37) with force-fit engagement.

14. Endoscopic suturing machine according to Claim 13, **characterized in that** the force-fit engagement is provided by a leaf spring or compression spring (33) bearing on the upper pressure piece (29).

15. Endoscopic suturing machine according to Claim 14, **characterized in that**, in order to modify its pressing force, the leaf spring or compression spring (33) is assigned an adjusting means (34), of which the handle (35) is arranged outside the housing (1).

16. Endoscopic suturing machine according to Claim 8, **characterized in that** the receiving device (17) is connected to the needle bar (5) by means of a connection piece (35), and the connection piece (35) at the same time serves for guiding the thread.

17. Endoscopic suturing machine according to Claim 16, **characterized in that** the connection piece (35) is arranged in relation to the receiving device (17) and the needle bar (5) in such a way that the thread can be inserted free of deflection from the receiving device (17) into the connection piece (35).

## Revendications

1. Machine à coudre endoscopique, présentant un boîtier (1) avec une partie supérieure de boîtier (3) pour recevoir des entraînements pour des outils de formation de points, une bobine de fil recevant une réserve de fil, une tige de boîtier se raccordant à la partie supérieure de boîtier pour recevoir des moyens de transfert des mouvements générés par les entraînements aux outils de formation de points, qui présentent au moins une barre d'aiguille (5) guidée dans la tige de boîtier avec une aiguille guidant le fil (4) et un crochet (6) agissant conjointement avec celle-ci pour former des points, qui est reçu par une barre du crochet (7),
**caractérisée en ce que**
la barre d'aiguille (5) pour recevoir et guider le fil est réalisée sous forme de corps creux et les éléments de guidage de fil (36, 8, 35) (36) (8) (35)) disposés entre la bobine de fil (23) recevant la réserve de fil et la barre d'aiguille (5) sont disposés sur la barre d'aiguille (5) de telle sorte que le fil s'étende sous forme rectiligne entre la sortie d'un dispositif de réception (17) pour la bobine de fil (23) et l'entrée dans la barre d'aiguille (5).

2. Machine à coudre endoscopique selon la revendication 1, **caractérisée en ce qu'**à l'intérieur de la barre d'aiguille (5) est disposé un élément de guidage (9) pour le fil, traversant celle-ci.

3. Machine à coudre endoscopique selon la revendication 1, **caractérisée en ce que** la barre d'aiguille (5) présente, dans la région des moyens de fixation pour l'aiguille (4), une ouverture de sortie (10) pour le fil orientée vers l'extérieur et de préférence obliquement vers le bas.

4. Machine à coudre endoscopique selon la revendication 1, **caractérisée en ce que** la barre d'aiguille (5) présente, dans la région des moyens de fixation pour l'aiguille (4), une rainure longitudinale (11) servant à recevoir le fil.

5. Machine à coudre endoscopique selon la revendication 4, comprenant une aiguille (4) présentant un piston plat avec une gorge de fil (12), **caractérisée en ce que** la rainure longitudinale (11) forme conjointement avec la gorge de fil (12) de l'aiguille (4) un canal de fil (13) et le fil ressort au niveau de son côté frontal ouvert hors du canal de fil (13).

6. Machine à coudre endoscopique selon la revendication 1, **caractérisée en ce que** l'aiguille (4) est réalisée sous forme de canule (15) et présente dans la région restante de l'oeillet un évidement latéral (16) pour la pointe du crochet (6).

7. Machine à coudre endoscopique selon la revendication 1, **caractérisée en ce que** la réserve de fil est reçue à l'intérieur d'un dispositif de réception (17) et celui-ci peut être connecté de manière amovible à la barre d'aiguille (5).

8. Machine à coudre endoscopique selon la revendication 7, **caractérisée en ce que** le dispositif de réception (17) pour la réserve de fil est formé par un boîtier en deux parties (18) dont la partie de base (19) présente un logement de forme cylindrique (21) avec un tourillon de centrage (22) pour la bobine de fil (23).

9. Machine à coudre endoscopique selon la revendication 7, **caractérisée en ce que** le diamètre intérieur du logement (21) pour la bobine de fil (23) est supérieur de seulement quelques dixièmes de millimètres au diamètre extérieur des brides (24) de la bobine de fil (23).

10. Machine à coudre endoscopique selon la revendication 8, **caractérisée en ce qu'**une surface d'appui (25) pour la bobine de fil (23) est réalisée au niveau du tourillon de centrage (22), laquelle présente un espacement axial par rapport à la surface de fond (26) du logement (21).

11. Machine à coudre endoscopique selon la revendication 1, **caractérisée en ce que** la barre d'aiguille (5) est disposée à l'intérieur d'un manchon (28) entraîné par une bielle (27) et peut être connectée de manière amovible au manchon.

12. Machine à coudre endoscopique selon la revendication 11, **caractérisée en ce que** la connexion entre le manchon (28) et la barre d'aiguille (5) est formée par une fermeture à baïonnette (20), dont la partie fixe (30) est connectée au manchon (28) et dont la partie mobile (31) est connectée à la barre d'aiguille (5).

13. Machine à coudre endoscopique selon la revendication 7, **caractérisée en ce que** le dispositif de serrage de fil (8) est disposé à l'intérieur du dispositif de réception (17) pour la réserve de fil, et est formé par deux pièces de pression (29, 37) disposées parallèlement l'une à l'autre et l'une au-dessus de l'autre, la pièce de pression supérieure (29) reposant par engagement par force sur la pièce de pression inférieure (37).

14. Machine à coudre endoscopique selon la revendication 13, **caractérisée en ce que** l'engagement par force est formé par un ressort à lame ou un ressort de compression (33) reposant sur la pièce de pression supérieure (29).

15. Machine à coudre endoscopique selon la revendication 14, **caractérisée en ce qu'**un moyen de réglage (34) est associé au ressort à lame ou ressort de compression (33) pour modifier sa force de compression, sa manette (35) étant disposée à l'extérieur du boîtier (1).

16. Machine à coudre endoscopique selon la revendication 8, **caractérisée en ce que** le dispositif de réception (17) est connecté au moyen d'une pièce de liaison (35) à la barre d'aiguille (5), et la pièce de connexion (35) sert simultanément au guidage du fil.

17. Machine à coudre endoscopique selon la revendication 16, **caractérisée en ce que** la pièce de connexion (35) est disposée par rapport au dispositif de réception (17) et à la barre d'aiguille (5) de telle sorte que le fil puisse être introduit sans déviation depuis le dispositif de réception (17) dans la pièce de connexion (35).
